# EUROPEAN PATENT APPLICATION

(11) **EP 1 512 750 A1**
(43) Date of publication of application: **09.03.2005**
(21) Application number: 03708474.6
(22) Date of filing: 28.02.2003
(51) Int. Cl.: C12P 19/44

(54) **PROCESS FOR PRODUCING FINE SELF-AGGREGATE**

(30) Priority: 07.03.2002 JP 2002061797
(71) Applicant: Japan Science and Technology Agency, Kawaguchi-shi, Saitama 332-0012 (JP); National Institute of Advanced Industrial Science and Technology, Tokyo 100-8921 (JP)
(72) Inventor: UZAWA, Hirotaka, Nat.Inst.Adv.Ind.Science&Techn., Tsukuba-shi, Ibaraki 305-8561 (JP); ZENG, Xiaoxiong, Nat.Inst.Adv.Ind.Sci&Technol., Tsukuba-shi, Ibaraki 305-8561 (JP); SHIMIZU, Toshimi, Nat.Inst.Adv.Ind.Sci&Technol., Tsukuba-shi, Ibaraki 305-8561 (JP); JOHN, George, c/o Nat.Inst.Adv.Ind.Sci & Technol., Tsukuba-shi, Ibaraki 305-8561 (JP); MINOURA, Norihiko, Nat.Inst.Adv.Ind.Sci & Technol., Tsukuba-shi, Ibaraki 305-8561 (JP)
(74) Representative: Wilson Gunn Gee
(86) International application number: PCT/JP2003/002298
(87) International publication number: WO 2003/074720

(57) **Abstract**

A novel saccharide chain is built on a surface by bonding a second saccharide to the glycolipid present on the surface of a self-aggregate under mild enzyme chemical reaction conditions.
1) A fine self-aggregate comprising an O-glycoside type glycolipid having the structure represented by the formula (chemical formula 2) below

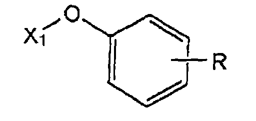

(in the formula X₁ represents a glycosyl group or an oligosaccharide group having two to twenty-nine monosaccharides and R is as defined above), 2) a monosaccharide or its derivative and 3) a glycotransferase or glycolytic enzyme are reacted in water.

## Description

### Technical Field

The present invention relates to a process for producing fine self-aggregates wherein enzyme chemistry is used to add an additional saccharide under mild conditions and in an aqueous solution to fine self-aggregates such as nanotubes and nanofibers formed by a voluntary aggregation of glycolipids.

### Prior Art

Cardanyl glucoside, a long chain alkyl phenol derivative having glucose substitution, is traditionally synthesized from a cardanol starting material that has been separated from natural plant materials and purified. When cardanyl glucoside is heated and dissolved in water and allowed to cool gradually, nanotube shaped aggregates are known to form through hydrogen bonding. [G. John, M. Masuda, Y. Okada, K. Yase and T. Shimizu, Adv. Mat., 13, 715 (2001)]

The inventors disclosed this type of self-aggregate production process (Japanese Patent Applications 2001-363762, 2002-35035 and the like) and a self-aggregate was obtained when using an oligosaccharide in the glyco chain (Japanese Patent Application 2002-49238). In these disclosures, glucose, galactose, mannose and other monosaccharides and lactose and other polysaccharides were used as saccharide chains, and the surface of the coagulated material was covered with uniform (identical). saccharides.

### Problems to be solved by the Invention

However, no nanotubes and nanofibers simultaneously containing two or more different types of saccharides or saccharide chains are known.

Previously, a so called solid phase synthetic method has been known as a method to introduce a saccharide chain onto a solid surface or to introduce another saccharide into an existing saccharide (for example, an article by Manabe and to published in Kobunshi, Volume 47, page 96, 1998). However, saccharide chains have not been introduced or modified using a solid phase synthetic method when self aggregates such as nanotubes and nanofibers having properties in between liquid crystals and crystals were involved. In addition, the solid phase synthetic method almost always utilized an organic chemistry approach.

### Means to solve the Problems

The present invention was developed in view of the current status of the conventional technology described above and presents an enzyme chemistry method to build a novel saccharide chain on a surface by bonding a second saccharide to the glycolipid present on the surface of a self-aggregate under mild conditions.

That is, the present invention is a process for producing a fine self-aggregate comprising an O-glycoside type glycolipid having a structure represented by the formula (chemical formula 1) below 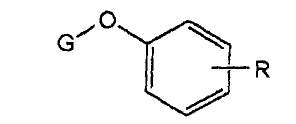 (in the formula, G represents an oligosaccharide group composed from two to thirty bonded monosaccharides, and R represents a hydrocarbon group having six to twenty-five carbon atoms) comprising a step of reacting 1) a fine self-aggregate comprising an O-glycoside type glycolipid having a structure represented by the formula (chemical formula 2) below 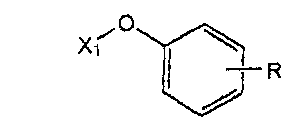 (in the formula, X₁ represents a glycosyl group or an oligosaccharide group having two to twenty-nine monosaccharides and R is as defined above), 2) a monosaccharide or its derivative and 3) a glycotransferase or glycolytic enzyme in water.

In this method, 1) a fine self aggregate comprising an O-glycoside type glycolipid having the construction represented by the formula (chemical formula 2) shown below 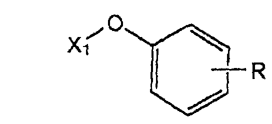 (in the formula, X₁ and R are as defined above), 2) a complex of monosaccharide and nucleic acid and 3) a glycotransferase may be reacted in water. Galactose transferase, sialyl transferase or fucosyl transferase are preferred examples of the glycotransferase, and reaction systems also containing a protein are preferred.

In addition, 1) a fine self aggregate comprising an O-glycoside type glycolipid having the construction represented by the formula (chemical formula 2) shown below 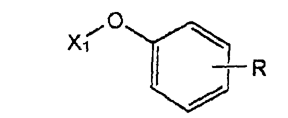 (in the formula, X₁ and R are as defined above), 2) a material linked with aryl groups and monosaccharide groups that may be substituted and 3) a glycotransferase may be allowed to react in water in the method described above. α-Galactosidase, β-galactosidase, sialydase, α-glucosidase or β-glucosidase is a preferred example of this glycolytic enzyme.

In addition, the saccharides may be introduced in multiple stages in these production processes by conducting said step multiple times.

### Brief description of the drawings

Figure 1 shows a fluorescent microscope photograph of a material obtained by bonding a galactose introduced into the glucose groups present on nanofiber surfaces and a fluorescent labeled lectin (75 µm vertical x 100 µm horizontal).
Figure 2 shows a fluorescent microscope photograph of a material obtained by bonding a galactose introduced into the glucose groups present on nanofiber surfaces and an FITC labeled lectin (75 µm vertical x 100 µm horizontal).

### Detailed description of the Invention

The compound constituting the self-aggregate targeted for a treatment in the present invention is an O-glycoside type glycolipid represented by the formula (Chemical formula 2) shown below.

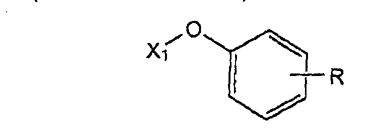

In the present invention, the hydrocarbon group (R) may be located o-, m- or p- to the -O-X₁ group, but the meta (m-) position is preferred.

In said formula (Chemical formula 2), X₁ is a glycosyl group or an oligosaccharide group linked with two to 29 monosaccharides, preferably is a glycosyl group or an oligosaccharide groups linked with 2-5 monosaccharides and more preferably is a glycosyl group. As examples of the group, aldopyranoses such as glucopyranose, galactopyranose, mannopyranose, allopyranose, altropyranose, gulopyranose, idopyranose and talopyranose and corresponding aldopyranoses from which hydrogen atom had been removed from the reducing terminal hydroxyl groups; commercially available compounds such as lactose, melibiose, cellobiose and galactosyl-α(1→4) galactose; and oligosaccharides obtained using chemical or enzymatic syntheses may be cited. The anomer glycoside linkage in a reducing terminal may be any one of α-anomer, β-anomer and a mixture thereof.

Simultaneously, hydrocarbon groups having six to 25 carbon atoms, preferably having fourteen to sixteen carbon atoms, more preferably having fifteen carbon atoms may be used as the R in said formulas (Chemical formula 1 and 2), and the R is an aliphatic hydrocarbon comprising saturated or unsaturated aliphatic hydrocarbons having one to five double bonds, preferably unsaturated aliphatic hydrocarbons having one to three double bonds. As examples of these hydrocarbon groups, the dodecyl group, the tridecyl group, the tetradecyl group, the pentadecyl group, the hexadecyl group, the heptadecyl group, the octadecyl group and these groups having monoene, diene, triene and the like as unsaturated bonds, for example, may be mentioned. However, the 8-pentadecenyl group, the 8,10-pentadecadienyl group and the 8,10,12-pentadecatrienyl group are preferred based on ready raw material availability.

The O-glycoside type glycolipids represented by said formula (chemical formula 2) can be manufactured, for example, using the method shown below.

Sugar substituted long chain hydrocarbon derivatives of phenols can be obtained by adding an oligosaccharide having hydroxyl groups all protected using acetyl groups to a long chain hydrocarbon substituted phenol represented by the formula (chemical formula 3) 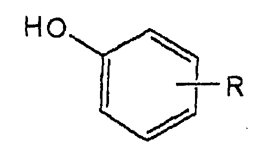 (in the formula R is as defined above) to glycoside bond to the hydroxyl group segment followed by the removal of the protective acetyl groups in the saccharide group.

An example of the acetylation procedure is described as follows. An oligosaccharide is dissolved in dry pyridine, acetic anhydride is added and the mixture is agitated using a magnetically driven stirrer for an hour to overnight at room temperature to 40°C. At this point, the reaction proceeds rapidly when dimethyl aminopyridine is added to the reaction system. Pyridine is removed using azeotropic distillation repeated five to seven times using a mixed solvent of toluene and ethanol, and the residue is dissolved in chloroform and is washed several times using a saturated aqueous sodium bicarbonate solution. The solution is concentrated, purified using a silica gel column, concentrated and dried.

The fine self aggregate production process is not particularly restricted in the present invention, but an aggregate may be obtained by dispersing the O-glycoside type glycolipid described above in water, heating the dispersion using a mantle heater, allowing the mixture to boil for about twenty minutes, allowing it to naturally cool to room temperature and allowing it to stand at room temperature to form a fine self-aggregate (Japanese Patent Applications 2000-271192 and 2001-363762).

Next, an example of the fine self-aggregate synthesis method of the present invention is explained.

First, a fine self-aggregate (substrate) is allowed to form as described above. A donor containing monosaccharide groups is used in excess of the substrate (for example, from an equal weight to 100 times the weight) in HEPES or phosphoric acid buffer in the presence of a glycotransferase or glycolytic enzyme, preferably in the presence of glycotransferase, and the reaction mixture is allowed to react by gently shaking it or by leaving it undisturbed at room temperature to 50°C, preferably at about 35°C, for one minute to 30 days.

The concentration of the HEPES or phosphoric acid buffer solution is preferably 0.01 mM to 10 mM and more preferably 10 mM to 100 mM, and the pH is preferably 4 to 8 and more preferably 6.5 to 7.8.

The amount of enzyme may be a catalytic amount or an excess (for example, a 100 fold excess) to the substrate, and from 1 mU to 10,000,000 U and preferably 10 mU to 1 U in terms of units.

This reaction is different depending on the enzyme used. Each form is explained.
(1) A case in which a glycotransferase is used.
   The substrate mentioned above, 1), a complex of monosaccharide and nucleic acid, 2), and a glycotransferase, 3), are allowed to react in water.
   UDP, CMP and the like are used as the nucleic acid, and the selection is made upon considering the enzyme reactivity. The complex contains a link between the phosphoric acid segment of the nucleic acid and, mainly, the position one hydroxyl group of the monosaccharide and may also contain a phosphoric acid section linked to an alkali metal such as sodium. In addition, a catalytic amount (for example, 1% by weight to 10% by weight based on UDP-sugar) of Mn²⁺ (for example, MnCl₂) may also be added when necessary. As examples of such a complex, UDP-sugar (for example, uridine 5'-diphosphogalactose), CMP-sugar (for example, cytidine 5'-monophospho-N-acetylneuraminic acid) and the like may be mentioned.
   As glycotransferase, galactose transferase [International Union of Biochemistry (IUB) Enzyme Committee Report number: EC. 2.4.1.22), sialyl transferase (EC. 2.4.99.1), fucosyl transferase (EC. 2.4.1.69) and the like may be mentioned.
   When using a glycotransferase, an enzyme such as alkaline phosphatase capable of decomposing the diphosphoric acid derivatives formed during the reaction is sometimes needed (the amount used is 1 mU to 10,000 U or preferably 10 mU to 10 U). When a second saccharide is introduced into the glycolipid (for example, glucose) in a target substrate, the co-presence of a protein such as lactoalbumin or albumin (ordinarily at least in equimolar amount to the glycotransferase) is sometimes needed.
   This protein is an optional ingredient that forms a complex with an enzyme in a reaction solution and is thought to play a role in transforming a saccharide supply source to a saccharide group on the surface of a fine self-aggregate. When this protein is absent, the transfer efficiency is sometimes reduced extensively. [Schanbacher, F. and Ebner, K.E., J. Biol. Chem., Vol. 245, pp. 5057 (1970), Brew, K., et. al., Proc. Natl. Acad. Scie. USA, Vol. 59, pp. 491 (1968), Fitzgerald, D.K., et. al., J. Biol. Chem., Vol. 245, pp. 2103 (1970), Narimatsu, H., et. al., Proc. Natl. Acad. Sci. USA, Vol. 83, pp. 4720 (1986)]
(2) A case in which a glycolytic enzyme is used.

The substrate mentioned above, 1), a bonded material of an aryl group that may be substituted and a monosaccharide group, 2) and a glycolytic enzyme, 3) are allowed to react in water.

An aryl group activates a saccharide group upon bonding with a saccharide group and is thought to enhance the receptivity for a glycolytic enzyme attack. A phenyl group is the preferred aryl group. This aryl group may contain a substituent, for example, a nitro group to reinforce this function.

As such a bonded material, saccharides activated by introducing an aromatic group into a saccharide aglycon segment such as 4-nitrophenyl saccharides (for example, 4-nitrophenyl β-D-galactopyranoside), 2-nitrophenyl saccharides (for example, 2-nitrophenyl β-D-galactopyranoside), and umbelliferyl saccharides (for example, umbelliferyl β-D-galactopyranoside), for example, may be mentioned. The reaction may also be conducted using a free saccharide itself or a saccharide, a different saccharide, (for example, lactose and the like).

Such a bonded material or saccharide is decomposed by a glycolytic enzyme, and the decomposed saccharide group is bonded to a saccharide substrate (an earlier formed fine self-aggregate).

As a glycolytic enzyme, α-galactosidase (EC. 3.2.1.22), β-galactosidase (EC. 3.2.1.23), sialidase (EC. 3.2.1.18), α-glucosidase (EC. 3.2.1.20), β-glucosidase (EC. 3.2.1.21) and the like, for example, may be mentioned.

The preferred reaction time for such enzymatic reactions is 24 hours to 240 hours. An enzyme and a donor may be added in several stages. Furthermore, the pH of a reaction system declines (favors the acidic side) as the reaction progresses, and the use of an aqueous base solution such as 0.1N KOH or NaOH to neutralize the system is desirable. The pH is preferably controlled to avoid exceeding 7.8.

The product is purified after the reaction by centrifugally separating it for one minute to one hour at 500 rpm to 20,000 rpm. The product is washed several times using the same buffer solution or distilled water in order to remove the unreacted reagent,.

A third saccharide in addition to the second saccharide already present can be introduced by repeating the process described above on the nanotubes and nanofibers into which the second saccharide had been introduced. By repeating the process, n number of saccharides can be successively introduced and nanotubes and nanofibers having saccharide chain surfaces which have been modified can be constructed.

In order to evaluate the saccharide chain introduction ratio, the nanotubes and nanofibers may be completely hydrolyzed (80°C to 100°C for ten minutes to five hours) to the Individual saccharides and alcohols in the aglycon segments upon conclusion of the enzyme reaction using a strong acid such as 0.1 M to 1.0 M trifluoroacetic acid. Here, the fat soluble, long chain aromatic alcohol derived from the aglycon is removed, and the remainder is converted to fluorescence labeled individual saccharide derivatives by allowing the water soluble constituent saccharides to react with a fluorescence developing group or an ultraviolet visible color developing group such as ethyl 4-aminobenzoate ester in an acetic acid-BH₃ 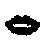 pyridine complex for ten minutes to three hours at 50°C to 100°C. Next, galactose, mannose, glucose and the like are eluted when an HPLC analysis is conducted using a reverse phase column (C-18 and the like) and 0.2M potassium borate/acetonitrile (93/7, v/v) buffer (pH 8.9) or 0.02% TFA/acetonitrile (1:1, v/v) as the elution solution at room temperature to 40°C. The proportion of individual constituent saccharides can be calculated using the peak areas. A fluorescence monitor at Ex. 305 nm, Em. 360 nm and a UV monitor at 305 nm are preferred. The second saccharide introduction ratio is ordinarily about 1% to 30%. In addition, a saccharide used as a standard is preferably converted simultaneously to its derivatives along with a sample using the same method described above in the HPLC analysis, and a calibration line is preferably prepared.

A fluorescence labeled lectin may also be utilized in addition to the HPLC analysis described above as an analytical method for the newly introduced saccharides. Standard lectins useful as the lectin in this method for the recognition of the saccharide introduced are commercially available. For example, concanavalin A derived lectin (henceforth referred to as Con A) for glucose and Erythrina derived lectin (henceforth referred to as ECA) for galactose are available. In addition, FITC recognizing, TRITC recognizing, Texas Red and the like are available commercially as forms bonded with lectins as fluorescent color developing groups.

Sugar groups are confirmed using the following procedure with fluorescence labeled lectins such as those mentioned above. From an equimolar amount to 100 times the equimolar amount of a commercially available fluorescence labeled lectin is added based upon the anticipated amount of saccharide groups in the nanotubes and nanofibers (substrates) formed through self aggregation of the glycolipids described above in an HEPES buffer solution or a phosphoric acid buffer solution (both having a concentration of 0.01 mM to 10 mM but 10 mM to 100 mM is ordinarily preferred). (The pH of the solution is four to eight, but a pH of 6.5 to 7.8 is preferred.) Several types of lectins having different equimolar amounts are ordinarily prepared, and the best suited variety is selected. When Con A is used as the lectin, manganese ion is needed in addition (ordinarily 1 mM to 100 mM). The lectin is allowed to penetrate gradually over 30 minutes to two days at 0°C to 40°C and is allowed to react by letting it stand. The system is ordinarily allowed to stand for twenty hours at room temperature.

The "post treatment" used upon completion of the reaction is the same method as previously described above. In addition, the fluorescence labeling of said fibers and tubes is examined under a fluorescence microscope.

A lectin having no capacity to bond with saccharide should be used in as a control, and the absence of bonding needs to be confirmed. Furthermore, interference experiments may also be conducted when needed. That is, a saccharide identical to the one present on the surface of the nanotubes and nanofibers is added in an amount from equimolar to a 100 fold excess. A fluorescence labeled lectin is added, and the absence of labeling on said fibers and tubes is preferably confirmed.

The presence of saccharide groups may also be directly confirmed using lectins such as fine metal colloidal particles under electron microscopy (TEM, SEM and the like) when necessary. The experimental procedure is as described above. In addition, it is preferred that the reference and interference experiments described above be conducted when needed.

### Effect of the Invention

The structure of a fine self-aggregate of the present invention is not stable at temperatures above 46°C in the presence of an organic solvent, and it is present in a stable form in water only at temperatures below 46°C. [Cf. G. John, M. Masuda, Y. Okada, K. Yase, T. Shimizu, Advanced Materials, Vol. 13, pp. 715 (2001).] This means that various reagents for organic synthesis are used to introduce a saccharide in an organic solvent or an enzyme and mild conditions are used to introduce a saccharide in an aqueous solution in which said nanotubes can stably exist when, for example, said nanotubes need to be modified. In the former case, the operations are difficult to conduct due to the reasons mentioned above. According to the method of the present invention, a new saccharide or a saccharide chain can be introduced under conditions as mild as biological reactions using enzyme chemistry in water or a buffer solution having suppressed ionic strength without using any organic solvent.

Furthermore, additional saccharide chains can be linked to the saccharide chains of fine self-aggregates such as nanofibers formed according to a method of the present invention. For example, a pathogenic virus such as an influenza virus may be captured and neutralized if a natural ligand saccharide chain such as NeuAc α2-3 (or 6) Gal β1-4 GlcNAc (Y. Suzuki et. al., J. Biol. Chem., Vol. 261, pp. 17057-17061) that is capable of bonding with the influenza virus can be bonded using an α2→6 bond. Furthermore, pathogenic coli O-157 itself or the Bello toxin produced by it can be effectively bonded if a saccharide chain constructed with a Gal α1-4Gal structure capable of bonding with O-157 or the Bello toxin produced by it can be introduced using an α1-4 bond [K. Karlsson et. al., J. Biol. Chem., Vol. 260, pp. 8545 (1985).] in this manner, nanotubes and nanofibers having unique bonding properties for specific toxic viruses, pathogenic bacteria, toxins and the like can be made available if saccharide chains (referred to as ligand glycochains) having suitable orientations can be introduced.

The present invention will be illustrated by the examples, but it is not intended to restrict the scope of the invention.

### Production Example 1

Cashew nut oil was vacuum distilled twice at about 400 Pa, and the fraction boiling from 220°C to 235°C was collected to obtain cardanol. The cardanol obtained, 1.52 g (five millimoles) was dissolved in 10 ml of anhydrous methylene chloride, and 3.9 g (five millimoles) of β-D-glucose penta-acetate and 0.62 ml (five millimoles) of boron trifluoride diethyl ether were added in the presence of 2 g of molecular sieve A. The reaction mixture was first agitated for 24 hours at room temperature and then poured into a 5% aqueous sodium bicarbonate solution. The organic phase was separated, washed with aqueous sodium bicarbonate solution followed by water and dried over anhydrous sodium sulfate. The organic solvent was completely removed using distillation, and the crude product obtained was recrystallized from ethanol. The solid product obtained was subjected to column chromatography using hexane/ethyl acetate (volume ratio 7/3) as the eluant, and 2.36 g (75% yield) of white solids of 1-(O-β-D-glucopyranoside tetra-acetate) cardanol was obtained.

The physical properties of this product are presented below.
Thin layer chromatography Rf value: Rf₁ = 0.47
Melting point: 60°C
Elemental analysis (C₃₅H₅₀O₁₀)

| | C | H |
|---|---|---|
| Calculated values (%) | 66.65 | 7.99 |
| Experimental values (%) | 66.78 | 7.82 |

Next, a 45% by mass aqueous trimethylamine solution was mixed with four times its volume of methyl alcohol and was allowed to react for 24 hours with the 1-(O-β-D-glucopyranoside tetra-acetate) cardanol previously obtained. The solvent was removed under reduced pressure, and the syrup-like residue obtained was crystallized from a mixed solvent of methyl alcohol/acetonitrile (volume ratio 1/2). The product was recrystallized from the same solvent to obtain 0.88 g (95% yield) of white solids of the desired deacetylated 1-(O-β-D-glucopyranoside) cardanol (referred to as "cardanyl glucoside) almost quantitatively.

The physical properties of the product are as follows.
Melting point: 132.5°C
Elemental analysis (C₂₇H₄₂O₆)

| | C | H |
|---|---|---|
| Calculated values (%) | 70.10 | 9.15 |
| Experimental values (%) | 70.39 | 9.44 |

Saturated cardanyl β-D-glucopyranoside was obtained by conducting a catalytic hydrogenation of the cardanyl glucoside in methanol (Wako Junyaku) under a hydrogen stream in the presence of palladium (Kojima Kagaku).

### Production Example 2

The saturated cardanyl β-D-glucopyranoside (2.5 mg) obtained in Production Example 1 was added to a pressure resistant glass autoclave (Taiatsu Glass Kogyo K.K., 200 ml capacity hyper cluster TEM-V100) containing 100 ml of super pure water (Milli Q), and the autoclave was sealed after the oxygen gas in the vessel and dissolved oxygen were removed by purging with N₂ gas. The contents were heated to 115°C (dispersion temperature) at a rate of 2°C/minute with agitation. The added pressure at this dispersion temperature was 0.12 MPa. The solution was maintained at this temperature for 20 minutes (dispersion time) and was left standing at room temperature (25°C) after having been allowed to cool unaided to room temperature by cutting electrical power to the heater (cooling rate 1.6°C/minute). The solution was maintained at this temperature for three days after which cotton like floaters (nanofibers) could be observed in the solution.

### Example 1

Three milligrams of the nanofibers obtained in Production Example 1 were suspended in 10 mM of HEPES buffer solution (Kanto Kagaku K.K., 18356-40, pH 7.4, 0.4 ml), and 4 mg of UDP-α-D-galactopyranoside disodium salt (CalBioChem, 670111), 0.4 mg of α-lactoalbumin (Sigma, L5385), 15 mU of β-1,4-galactosyltransferase (CalBioChem, 345649), 5 mM of MnCl₂ (Kanto Kagaku, 25009-30) and 2 U of alkali phosphatase (Wako Junyaku K.K., 016-14631) were added before incubating the reaction mixture for 24 hours at 25°C. The reaction mixture was centrifuged at 6,400 rpm (for about ten minutes using Millipore Chibetan), the supernatent solution was discarded, all of the buffer solutions and reagents listed above were added to the remainder and the mixture was incubated at the same temperature for the same duration of time. The procedure was repeated a total of three times. Using this procedure, galactose was introduced to the glucose groups present on the nanofiber surface.

Here, the UDP-α-D-galactopyranoside disodium salt was the source of galactose, a monosaccharide added, β-1,4-galactosyltransferase was a transferase and α-lactoalbumin formed a complex with the enzyme in the reaction solution thought to play the role of selectively transferring galactose to the glucose groups present on the nanofiber surface.

Next, an ABEE saccharide chain identification kit (Seikagaku Kogyo K.K., 400871) was used to measure the saccharide addition ratio in order to confirm that galactose had been introduced to the glucose groups present on the nanofiber surface.

Three hundred micrograms of the sample mentioned above were added to 10 µl of 8M TFA, and the mixture was heated for three hours at 100°C. The mixture was centrifuged (for about ten minutes) at 6,400 rpm, and the residue was heated and dried at 100°C. The solid residue was dissolved in 40 µl of 2-propanol and concentrated again under reduced pressure. A fluorescence marker of 4-aminobenzoyl ethyl ester that had first been dissolved in methanol was added to the reside to provide 16.8 mg of the ester, and acetic acid and a pyridine 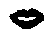 BH₃ complex were added to provide 170 µl and 3.58 mg, respectively, before allowing the mixture to react for 60 minutes at 80°C. After the reaction, the reaction mixture was allowed to stand at room temperature, and 200 µl of distilled water and chloroform were added. The solution was separated, centrifuged at 1,000 rpm, and the aqueous solution fractions were subjected to high performance liquid chromatography (HPLC).

Honenpack C18 (Honen K.K. 80045, 75 mm x 4.6 mm i.d.) was used as the column in the HPLC analysis and was eluted at a rate of 1.0 ml/minute at 40°C. The results were monitored at Ex. 305 nm, Em. 360 nm. The results were simultaneously monitored at 305 nm UV. A 0.2 M potassium borate buffer solution (pH 8.9) containing 7% acetonitrile was used as the eluant. The results are shown in Table 1.

| Retention time (minutes) | Relative area (%) | Attribution |
|---|---|---|
| 7.0 | 6.6 | Gal |
| 8.9 | 14.8 | Man isomerized from Glc |
| 12.0 | 78.6 | Glc |

Based on the results, the nanofiber contained 6.6% of newly introduced galactose. The balance (93.4%) was glucose already present on the nanofiber surface.

Next, labeled lectin was bonded to the galactose and examined microscopically to directly confirm the addition of galactose to the nanofiber surface.

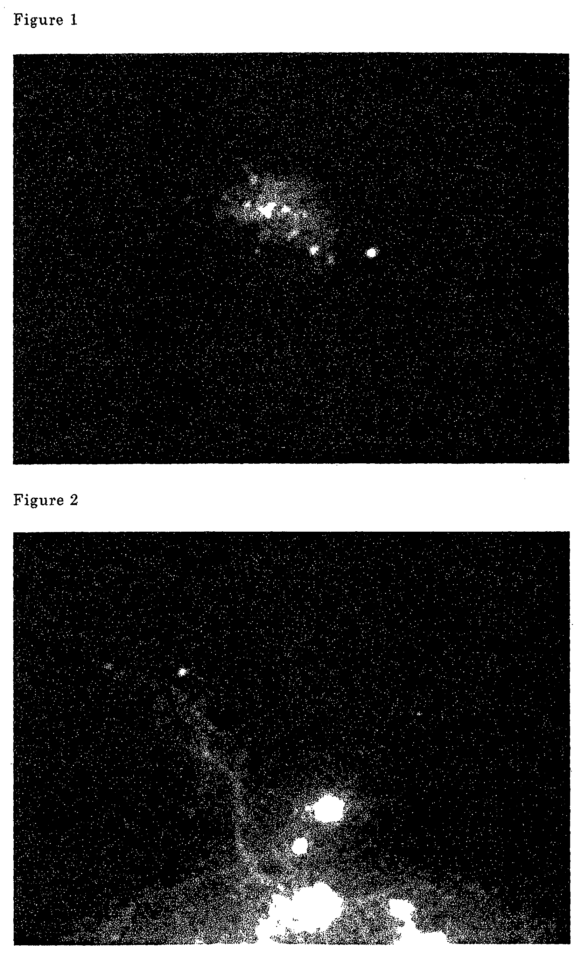

The fluorescence labeled lectin [Sigma Co. L3391, was an FITC labeled Erythrina derived lectin and was a fluorescence labeled protein containing a bonding capability (affinity) for β-galactose groups], 0.1 mg, was dissolved in 0.5 ml of 10mM HEPES (pH 7.4) buffer solution. Next, about 0.1 mg of said nanofiber prepared as described above was treated several times using the same buffer solution, and 300 µl of the fluorescence lectin solution previously used was added. The mixture was left standing for eighteen hours and centrifuged as described above, and 200 µl of the same HEPES buffer solution was added to the residue which was then examined using a fluorescence microscope and an ordinary optical microscope. (See Figure 1.)

The same examination described above was conducted using a different FITC labeled lectin (ConA, Sigma Co. C7642) uniquely capable of recognizing a glucose group. (See Figure 2)

Fibrous images were observed providing direct confirmation that galactose was introduced to the glucose groups present on the nanofiber surfaces.

## Claims

1. A process for producing a fine self-aggregate comprising an O-glycoside type glycolipid having a structure represented by the formula (chemical formula 1) below 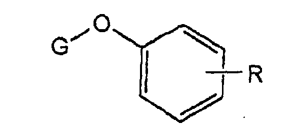 (in the formula, G represents an oligosaccharide group composed from two to thirty bonded monosaccharides, and R represents a hydrocarbon group having six to twenty-five carbon atoms) comprising a step of reacting 1) a fine self-aggregate comprising an O-glycoside type glycolipid having a structure represented by the formula (chemical formula 2) below 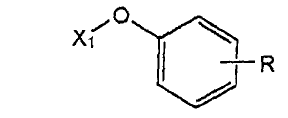 (in the formula, X₁ represents a glycosyl group or an oligosaccharide group having two to twenty-nine monosaccharides and R is as defined above), 2) a monosaccharide or its derivative and 3) a glycotransferase or glycolytic enzyme in water.

2. The process as in claim 1 wherein the step is reacting 1) a fine self-aggregate comprising an O-glycoside type glycolipid having a structure represented by the formula (chemical formula 2) below 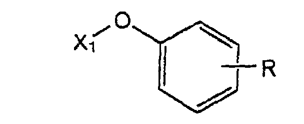 (in the formula X₁ and R are as defined above), 2) a complex of a monosaccharide and a nucleic acid and 3) a glycotransferase in water.

3. The process as in claim 2 wherein said glycotransferase is galactose transferase, sialyl acid transferase or fucosyl transferase.

4. The process as in claims 2 or 3 wherein the reaction system further contains a protein.

5. The process as in claim 1 wherein said step is reacting 1) a fine self-aggregate comprising an O-glycoside type glycolipid having a structure represented by the formula (chemical formula 2) below 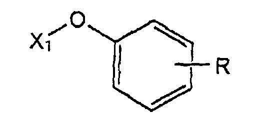 (in the formula X₁ and R are as defined above), 2) a bonded material of an aryl group that may be substituted and a monosaccharide group and 3) a glycolytic enzyme in water.

6. The process as in claim 5 wherein said glycolytic enzyme is α-galactosidase, β-galactosidase, sialydase, α-glucosidase or β-glucosidase.

7. The process as in claim 5 or 6 wherein said aryl group is a phenyl group.

8. The process as in any one of claims 1 to 7 wherein said step is conducted multiple times.

9. The process as in any one of claims 1 to 8 wherein a hydrocarbon group (R) is in a meta position to the -O-G group in said formula (chemical formula 1) and a hydrocarbon group (R) is in a meta position to the -O-X₁ group in said formula (chemical formula 2).
